# EUROPEAN PATENT SPECIFICATION

(11) **EP 0 507 484 B1**
(45) Date of publication and mention of the grant of the patent: **26.06.1996**
(21) Application number: 92302487.1
(22) Date of filing: 23.03.1992
(51) Int. Cl.: A61K 47/44

(54) **A pharmaceutical formulation of aureobasidins**
Aureobasidin enthaltendes Arzneimittel
Composition pharmaceutique à base d'aureobasidine

(30) Priority: 02.04.1991 JP 94954/91
(43) Date of publication of application: 07.10.1992
(73) Proprietor: TAKARA SHUZO CO. LTD., Fushimi-ku Kyoto 612 (JP)
(72) Inventor: Takesako, Kazutoh, Kusatsu-shi, Shiga-ken (JP); Yoshikawa, Yoshie, Kyoto-shi, Kyoto-fu (JP); Kuroda, Hiroyuki, Koga-gun, Shiga-ken (JP); Kato, Ikunoshin, Uji-shi, Kyoto-fu (JP); Inouye, Kazuhiro, Yokohama-shi, Kanagawa-ken (JP)
(74) Representative: Marlow, Nicholas Simon

(56) References cited:
- EP-A- 0 352 092

SEQUENCE LISTING
(1) GENERAL INFORMATION:
(i) APPLICANT: Kazutoh TAKESAKO et al.
(ii) TITLE OF THE INVENTION: Pharmaceutical Formulation of
Aureobasidins
(iii) NUMBER OF SEQUENCE: 2
(2) INFORMATION FOR SEQ ID NO:1:
(i) SEQUENCE CHARACTERISTICS:
(A) LENGTH: 8 amino acids
(B) TYPE: amino acid
(C) STRANDEDNESS: single
(D) TOPOLOGY: circular
(vi) ORIGINAL SOURCE:
(A) ORGANISM: Aureobasidium
(ix) FEATURE:
(A) NAME/KEY: modified-site
(B) LOCATION: 1
(C) IDENTIFICATION METHOD:
(D) OTHER INFORMATION: /note="MeVal"
(ix) FEATURE:
(A) NAME/KEY: modified-site
(B) LOCATION: 2
(C) IDENTIFICATION METHOD:
(D) OTHER INFORMATION: /note="Phe, o-fluorophenylalanine,
m-fluorophenylalanine, or Tyr"
(ix) FEATURE:

(A) NAME/KEY: modified-site

(B) LOCATION: 3

(C) IDENTIFICATION METHOD:

(D) OTHER INFORMATION: /note="N-methylphenylalanine, β-

hydroxy-N-methylphenylalanine, Phe, o-fluoro-N-

methylphenylalanine, m-fluoro-N-methylphenylalanine, N-

methyltyrosine, sarcosine, N-methylserine, β-oxo-N-

methylphenylalanine, or β-acyloxy-N-methylphenylalanine (wherein

the acyl group is a lower acyl group having 1 to 4 carbon atoms)"

(ix) FEATURE:

(A) NAME/KEY: modified-site

(B) LOCATION: 4

(C) IDENTIFICATION METHOD:

(D) OTHER INFORMATION: /note="Pro, 4Hyp, or thioproline"

(ix) FEATURE:

(A) NAME/KEY: modified-site

(B) LOCATION: 5

(C) IDENTIFICATION METHOD:

(D) OTHER INFORMATION: /note="aIle, Val, Leu, or Nle"

(ix) FEATURE:

(A) NAME/KEY: modified-site

(B) LOCATION: 6

(C) IDENTIFICATION METHOD:

(D) OTHER INFORMATION: /note="MeVal or Val"

(ix) FEATURE:

(A) NAME/KEY: modified-site

(B) LOCATION: 7

(C) IDENTIFICATION METHOD:

(D) OTHER INFORMATION: /note="Leu or Nva"
(ix) FEATURE:
(A) NAME/KEY: modified-site
(B) LOCATION: 8
(C) IDENTIFICATION METHOD:
(D) OTHER INFORMATION: /note="β-hydroxy-N-methylvaline,
γ-hydroxy-N-methylvaline, MeVal, N,β-dimethylaspartic acid, β-
hydroxy-N-methylphenylalanine, N-methylphenylalanine, N-methyl-
2,3-didehydrovaline, N-methyl-3,4-didehydrovaline, or sarcosine,
wherein this amino acid has as a substituent either 2-hydroxy-3-
methylbutanoic acid or 2-hydroxy-3-methylpentanoic acid, which
substituent is bound to the amino acid of location 1 to form the
cyclic peptide."
(xi) SEQUENCE DESCRIPTION: SEQ ID NO:1:
Xaa Xaa Xaa Xaa Xaa Xaa Xaa Xaa
1               5

(3) INFORMATION FOR SEQ ID NO:2:
(i) SEQUENCE CHARACTERISTICS:
(A) LENGTH: 8 amino acids
(B) TYPE: amino acid
(C) STRANDEDNESS: single
(D) TOPOLOGY: circular
(vi) ORIGINAL SOURCE:
(A) ORGANISM: Aureobasidium
(ix) FEATURE:
(A) NAME/KEY: modified-site
(B) LOCATION: 1

(C) IDENTIFICATION METHOD:
(D) OTHER INFORMATION: /note="MeVal"
(ix) FEATURE:
(A) NAME/KEY: modified-site
(B) LOCATION: 3
(C) IDENTIFICATION METHOD:
(D) OTHER INFORMATION: /note="N-methylphenylalanine"
(ix) FEATURE:
(A) NAME/KEY: modified-site
(B) LOCATION: 5
(C) IDENTIFICATION METHOD:
(D) OTHER INFORMATION: /note="aIle"
(ix) FEATURE:
(A) NAME/KEY: modified-site
(B) LOCATION: 6
(C) IDENTIFICATION METHOD:
(D) OTHER INFORMATION: /note="MeVal"
(ix) FEATURE:
(A) NAME/KEY: modified-site
(B) LOCATION: 8
(C) IDENTIFICATION METHOD:
(D) OTHER INFORMATION: /note="β-hydroxy-N-methylvaline,
wherein this amino acid has as a substituent 2-hydroxy-3-
methylpentanoic acid, which substituent is bound to the amino acid
of location 1 to form the cyclic peptide."
(xi) SEQUENCE DESCRIPTION: SEQ ID NO:2:
Xaa Phe Xaa Pro Xaa Xaa Leu Xaa
1               5

## Description

### Field of industrial applications:

This invention relates to novel formulations of Aureobasidins that can be used as antifungal agents and the like.

### State of the prior art:

Aureobasidins, antibiotics with antifungal activity produced by a strain of an Aureobasidium species, referred to as antibiotics R106, have been disclosed in U.S. Patents 5057493, 5158876 and 5260214 (Application No. 07/379629, and their related compounds and derivatives have also been disclosed in the above US patents from U.S. Patent Application No. 07/379629, Japan Laid-Open Patent Application 3-44398, and U.S. Patent No. 5200505 (Application No. 07/643948).

The structure of Aureobasidins are represented by general formula [I]; 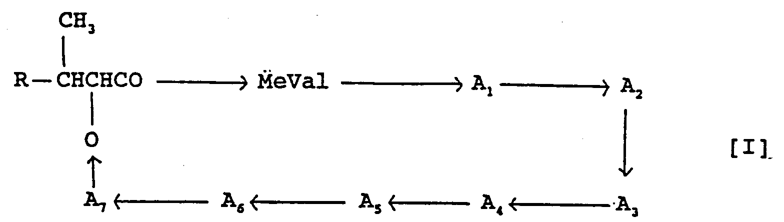
wherein:
R is methyl or ethyl;
A₁ is Phe, o-FPhe, m-FPhe, or Tyr;
A₂ is MePhe, β-HOMePhe, Phe, o-FMePhe, m-FMePhe, MeTyr, Sar, MeSer, β-oxoMePhe, or β-R₂OMePhe (wherein R₂ is a lower acyl group having 1 to 4 carbon atoms);
A₃ is Pro, 4Hyp, or SPro;
A₄ is allo-Ile, Val, Leu, or Nle;
A₅ is MeVal or Val;
A₆ is Leu or Nva;
A₇ is β-HOMeVal, γ-HOMeVal, MeVal, N,β-MeAsp, β-HOMePhe, MePhe, MeDH_{2,3}Val, MeDH_{3,4}Val, or Sar.

The compound with the strongest antifungal activity of all of these compounds has been named Aureobasidin A, and it has the structure shown below in formula [II]; 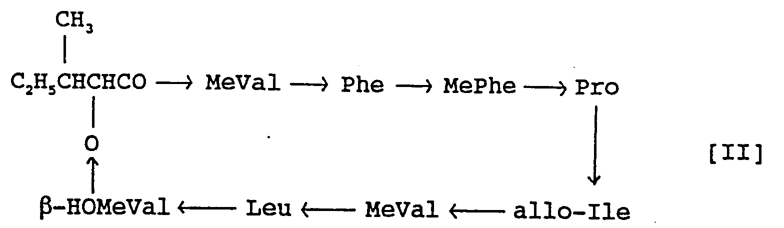
Abbreviations for amino acids used in the general formula described above are given below.
Phe: phenylalanine
o-FPhe: o-fluorophenylalanine
m-FPhe: m-fluorophenylalanine
MePhe: N-methylphenylalanine
β-HOMePhe: β-hydroxy-N-methylphenylalanine
β-R₂OMePhe: β-acyloxy-N-methylphenylalanine
β-oxoMePhe: β-oxo-N-methylphenylalanine
o-FMePhe: o-fluoro-N-methylphenylalanine
m-FMePhe: m-fluoro-N-methylphenylalanine
Tyr: tyrosine
MeTyr: N-methyltyrosine
Val: valine
MeVal: N-methylvaline
Nva: norvaline
β-HOMeVal: β-hydroxy-N-methylvaline
γ-HOMeVal: γ-hydroxy-N-methylvaline
MeDH_{2,3}Val: N-methyl-2,3-didehydrovaline
MeDH_{3,4}Val: N-methyl-3,4-didehydrovaline
allo-Ile: alloisoleucine
Leu: leucine
Nle: norleucine
N,β-MeAsp: N,β-dimethylaspartic acid
Pro: proline
4Hyp: 4-hydroxyproline
SPro: thioproline
Sar: sarcosine
MeSer: N-methylserine

### Problem to be solved by the invention:

Formulations in solid form are desired so that Aureobasidin A, its related compounds, and its derivatives (referred to hereafter as "Aureobasidins") can be used in clinical applications. The purpose of this invention is to provide formulations of Aureobasidins in solid form for use in clinical applications.

### Steps taken to solve the problem:

Briefly, this invention relates to a pharmaceutical formulation of Aureobasidins in solid form which comprises a pharmacologically effective amount of Aureobasidin and a nontoxic surface-active agent.

As the Aureobasidins of this invention, the compounds shown in the detailed descriptions of U.S. Patent Appl. No. 07/379629 (U.S. Patent 5260214), Japan Laid-Open Patent Application 3-44398, and U.S. Patent Appl. No. 07/643948 (U.S. Patent 5200505), which include Aureobasidin A, are included. For clinical use, Aureobasidin A is preferred.

The surface-active agents that can be used in this invention include ionic surface-active agents (cationic, anionic, or amphoteric; examples are alkyl sulfonate and benzalkonium chloride) and nonionic surface-active agents (examples are polyoxyethylene-hydrogenated castor oil, polyoxyethylenesorbit fatty acid esters, polyoxyethylenesorbitan fatty acid esters, polyoxyethyleneglycerol fatty acid esters, polyoxyethyleneglycol fatty acid esters, and polyoxyethylenealkyl phenyl ethers). For the surface-active agents used in this invention, nonionic surface-active agents are preferable, and among these are included, for example, polyoxyethylene-hydrogenated castor oil, such as Nikkol HCO-40, HCO-50, HCO-60 (Nikko Chemicals Co., Ltd.), and Uniox HC-40, HC-50, and HC-60 (Nippon Oil & Fats Co., Ltd.).

Of polyoxyethylenesorbit fatty acid esters, there are, for example, Nikkol GO-430, GO-440, GO-460, GL-1, Atlox 1045A, 1196, G-1045, and G-1441 (Kao Atlas Co., Ltd.). As polyoxyethylenesorbitan fatty acid esters, there are, for example, Tween 20, Tween 40, Tween 60, Tween 80, Emasol 1130, Emasol 3130, Nikkol TL-1010, TP-10, and TS-10. As polyoxyethyleneglycerol fatty acid esters, there are, for example, Nikkol TMGS-15 and TMGS-5. As polyoxyethyleneglycol fatty acid esters, there are, for example, Nikkol MYL-110 and MYS-10. As polyoxyethylenealkyl phenyl ethers, there are, for example, Nikkol NP-10 and Emulgen 810.

Of nonionic surface-active agents, polyoxyethylene-hydrogenated castor oil is particularly suitable, because compared with, for example, the polyoxyethylenesorbitan fatty acid ester Tween 80, it is more readily used for solid formulation, restrictions on its daily use are less, and its safety is greater.

In this invention, it is sufficient for the solid formulation to include at least one Aureobasidin and a surface-active agent, but it is also possible that a coating agent, a viscosity agent, a filler, and the like to be included as a carrier, and additives that are approved for use in pharmaceutical agents, such as stabilizers, coloring agents, fragrance agents, etc., may be included.

As a coating agent, the following may be used: hydroxypropylmethyl cellulose (for example, TC-5 from Shin-Etsu Chemical Co., Ltd.), polyvinylacetaldiethylamino acetate (AEA from Sankyo Co., Ltd.), hydroxypropyl cellulose (HPC-L from Nisso Co., Ltd.), aminoalkylmethacrylate copolymer E (Eudragit E100 from Röhm Pharma GmbH Weiterstadt), hydroxypropylmethyl cellulose phthalate (HPMCP from Shin-Etsu), hydroxypropylmethyl cellulose acetatesuccinate (AQOAT from Shin-Etsu), carboxymethylethyl celluose (CMEC OS and AQ from Freund Industrial Co., Ltd.), methacrylic acid copolymer L (Eudragit L100), methacrylic acid copolymer LD (Eudragit L30 D-55 ), and methacrylic acid copolymer S (Eudragit S100).

As a viscosity agent, there are, for example, glycerin, polyethylene glycol, polyglycerin, polyvinylpyrrolidone, polylactic acid, polyvinylalcohol, gelatin, hydroxypropyl starch, pullulan, carboxymethyl cellulose, sodium hyalurate, chitosan, and gum arabic.

As a shaping agent, there are, for example, D-mannitol, glucose, lactose, fructose, and other saccharides; dextran and other polysaccharides; α-cyclodextrin, β-cyclodextrin, γ-cyclodextrin, dextrinhydroxypropyl starch, and other dextrins; citric acid, maleic acid, tartric acid, malic acid, and other acids; urea and other neutral substances, lecithin, soya lecithin, and other phospholipids, and capric acid, myristic acid, and other middle-chain fatty acids.

The form of the formulation of this invention can be that of tablets, granules, soft capsules, hard capsules, drops, or any other solid form.

The formulations of this invention can be manufactured by any ordinary method for the formulation forms listed immediately above. For example, at least one Aureobasidin and a surface-active agent can be dissolved in an organic solvent such as ethanol, methanol, acetone, dimethylformamide, or chloroform, and mixed with, for example, a coating agent, if desired, after which the solvent is removed and the agent can be made into solid form, or, for example, an Aureobasidin can be added to a heated surface-active agent, after which a viscosity agent can be added and mixed in, after which the mixture can be solidified by being cooled.

The solid material obtained can be pulverized to give a powder. The powder can be made into granules in the usual way. For example, the powder can be mixed with a small amount of a solvent such as water, ethanol, or acetone, and the mixture can be passed through a mesh with holes of a suitable size to make granules, after which the granules can be dried. The proportions of the Aureobasidin and the surface-active agent may be in the ratio by weight of 1:0.01 to 1:100, preferably 1:1 to 1:10.

The solid formulation of Aureobasidins of this invention is suitable for clinical use and gives high concentrations in blood and various organs when taken orally.

Next, this invention will be explained by examples, but this invention is not to be taken to be restricted to these examples alone.

### Example 1.

First, 10 g of surface-active agent HCO-50 (Nikko Chemicals Co., Ltd.) was melted by being heated to 65°C, and to this, 10 g of Aureobasidin A and 100 g of polyethylene glycol 4000 were added. After the combination was thoroughly mixed, it was allowed to solidify at room temperature and granules were prepared.

### Example 2.

First, 50 g of surface-active agent HCO-50 (Nikko Chemicals Co., Ltd.) was melted by being heated to 65°C, and to this, 10 g of Aureobasidin A and 60 g of polyethylene glycol 4000 were added. After the combination was thoroughly mixed, it was allowed to solidify at room temperature and granules were prepared.

### Reference example 1.

First, 50 g of Aureobasidin A and 150 g of hydroxypropyl cellulose were dissolved in 95% (v/v) ethanol and the solvent was then removed under reduced pressure. The dry material obtained was pulverized to give a powder.

### Test Example 1.

To two groups of male Wistar rats five weeks old (with four rats in each group), the granular formulations obtained in Examples 1 and 2 were given orally at the dose of 40 mg/kg. The granules administered were 2 mm in diameter and 3 mm long. To the control group, the powder obtained in Reference Example 1 was administered in the same way. Blood was collected 1, 3, 6, and 8 hours after the administration, and the concentration of Aureobasidin A in the serum was assayed by high-pressure liquid chromatography. Results are in Table 1.

**Table 1**

| | Concentration in the serum (µg/ml) | | | |
|---|---|---|---|---|
| | 1 h | 3 h | 6 h | 8 h |
| Example 1 | 0.7±0.5 | 2.0±1.7 | 1.3±1.0 | 0.5±0.1 |
| Example 2 | 1.9±0.5 | 3.9±1.7 | 2.7±1.4 | 0.9±0.8 |
| Reference Example 1 | 1.0±0.6 | 1.5±0.8 | <0.2±0.3 | <0.1±0.2 |

| | | | | |
|---|---|---|---|---|
| *HPLC conditions: Column: Capcell Pak C18 6φ x 150 mm (Shiseido) Mobile phase: 80% acetontrile/20% water, 1.0 ml/min Detection: UV absorption at 220 nm | | | | |

As seen in Table 1, the formulations of this invention give higher concentrations of the agent in the serum than the formulation given to the control group.

As described above, by oral administration of the formulations of this invention, the concentrations of the agent in blood are high enough for pharmacological activity such as antifungal activity, so the formulations can be used in the treatment of fungal infections and the like.

Incidentally, the acute toxicity of Aureobasidins to be used in this invention, as orally administered, is LD₅₀ > 1000 mg/kg.

The dosage of the solid preparation of this invention may vary over a wide range depending upon the age, body weight, condition, etc. of the patient, but the usual dosage, as oral administration, is 2 - 500 mg/kg. day (calculated as Aureobasidin).

## Claims (Claims for the following Contracting State(s): AT, BE, CH, DE, DK, FR, GB, GR, IT, LI, LU, MC, NL, PT, SE)

1. A pharmaceutical composition in solid form comprising a pharmacologically effective amount of Aureobasidin represented by the general formula [I]; 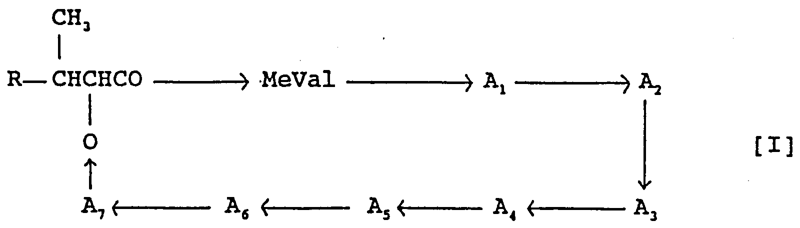 in which:
R is methyl or ethyl;
A₁ is Phe, o-FPhe, m-FPhe, or Tyr;
A₂ is MePhe, β-HOMePhe, Phe, o-FMePhe, m-FMePhe, MeTyr, Sar, MeSer, β-oxoMePhe, or β-R₂MePhe (wherein R₂ is a lower acyl group having 1 to 4 carbon atoms);
A₃ is Pro, 4Hyp, or SPro;
A₄ is allo-Ile, Val, Leu, or Nle;
A₅ is MeVal or Val;
A₆ is Leu or Nva; and
A₇ is β-HoMeVal, γ-HOMeVal, MeVal, N,β-MeAsp, β-HOMePhe, MePhe, MeDH_{2,3}Val, MeDH_{3,4}Val, or Sar, and a nontoxic surface-active agent.

2. A pharmaceutical composition according to claim 1, in which the surface-active agent is a nonionic agent.

3. A pharmaceutical composition according to claim 2, in which the nonionic agent is polyoxyethylene-hydrogenated castor oil.

## Claims (Claims for the following Contracting State(s): ES)

1. A pharmaceutical composition in solid form comprising a pharmacologically effective amount of Aureobasidin represented by the general formula [I]; 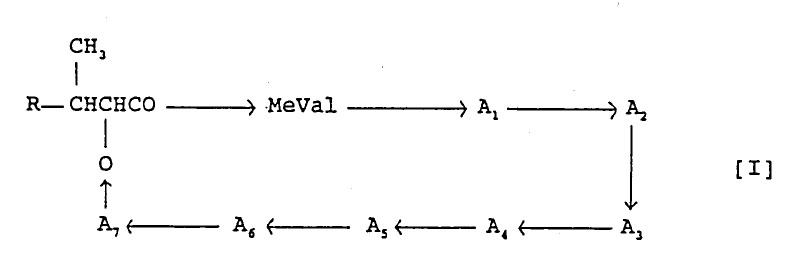 in which:
R is methyl or ethyl;
A₁ is Phe, o-FPhe, m-FPhe, or Tyr;
A₂ is MePhe, β-HOMePhe, Phe, o-FMePhe, m-FMePhe, MeTyr, Sar, MeSer, β-oxoMePhe, or β-R₂OMePhe (wherein R₂ is a lower acyl group having 1 to 4 carbon atoms);
A₃ is Pro, 4Hyp, or SPro;
A₄ is allo-Ile, Val, Leu, or Nle;
A₅ is MeVal or Val;
A₆ is Leu or Nva; and
A₇ is β-HOMeVal, γ-HOMeVal, MeVal, N,β-MeAsp, β-HOMePhe, MePhe, MeDH_{2,3}Val, MeDH_{3,4}Val, or Sar,
and a nontoxic surface-active agent.

2. A pharmaceutical composition according to claim 1, in which the surface-active agent is a nonionic agent.

3. A pharmaceutical composition according to claim 2, in which the nonionic agent is polyoxyethylene-hydrogenated castor oil.

4. Method of preparing a pharmaceutical composition in solid form comprising the step of adding a pharmacologically effective amount of Aureobasidin represented by the general formula [I]; 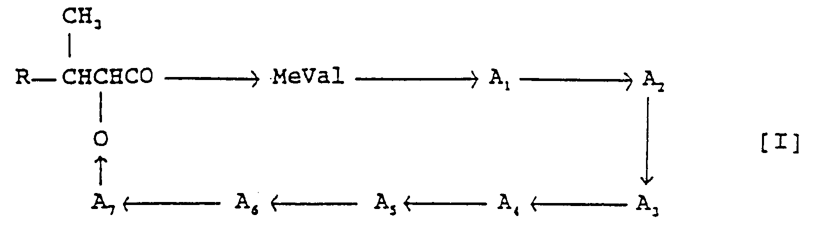 in which:
R is methyl or ethyl;
A₁ is Phe, o-FPhe, m-FPhe, or Tyr;
A₂ is MePhe, β-HOMePhe, Phe, o-FMePhe, m-FMePhe, MeTyr, Sar, MeSer, β-oxoMePhe, or β-R₂OMePhe (wherein R₂ is a lower acyl group having 1 to 4 carbon atoms);
A₃ is Pro, 4Hyp, or SPro;
A₄ is allo-Ile, Val, Leu, or Nle;
A₅ is MeVal or Val;
A₆ is Leu or Nva; and
A₇ is β-HOMeVal, γ-HOMeVal, MeVal, N,β-MeAsp, β-HOMePhe, MePhe, MeDH_{2,3}Val, MeDH_{3,4}Val, or Sar,
to a nontoxic surface-active agent.

5. Method according to claim 1 in which the surface-active agent is non-ionic agent

6. Method according to claim 2 in which the non-ionic agent is polyoxyethylene-hydrogenated caster oil.

## Patentansprüche (Patentansprüche für folgende(n) Vertragsstaat(en): AT, BE, CH, DE, DK, FR, GB, GR, IT, LI, LU, MC, NL, PT, SE)

1. Arzneimittel in fester Form enthaltend eine pharmakologisch wirksame Menge von Aureobasidin der allgemeinen Formel [I] 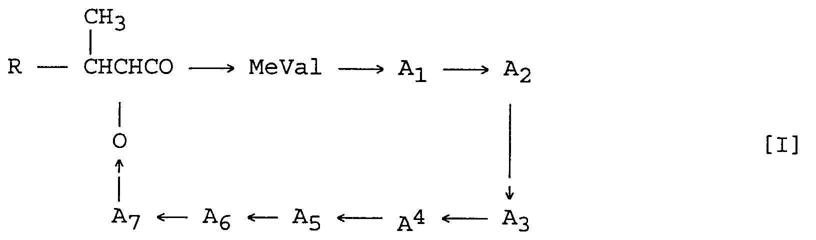 worin
R Methyl oder Ethyl,
A₁ Phe, o-FPhe, m-FPhe oder Tyr,
A₂ MePhe, β-HOMePhe, Phe, o-FMePhe, m-FMePhe, MeTyr, Sar, MeSer, β-oxoMePhe oder β-R₂OMePhe (worin R₂ eine niedere Acylgruppe mit 1 bis 4 Kohlenstoffatomen ist),
A₃ Pro, 4Hyp oder SPro,
A₄ allo-Ile, Val, Leu oder Nle,
A₅ MeVal oder Val,
A₆ Leu oder Nva, und
A₇ β-HOMeVal, γ-HOMeVal, MeVal, N,β-MeAsp, β-HOMePhe, MePhe, MeDH_{2,3}Val, MeDH_{3,4}Val oder Sar sind,
und ein nicht-toxisches oberflächenaktives Mittel.

2. Arzneimittel nach Anspruch 1, dadurch gekennzeichnet, daß das oberflächenaktive Mittel ein nicht-ionisches Mittel ist.

3. Arzneimittel nach Anspruch 2, dadurch gekennzeichnet, daß das nichtionische Mittel Polyoxyethylen-hydriertes Rizinusöl ist.

## Patentansprüche (Patentansprüche für folgende(n) Vertragsstaat(en): ES)

1. Arzneimittel in fester Form enthaltende ein pharmakologisch wirksame Menge von Aureobasidin der allgemeinen Formel [I] 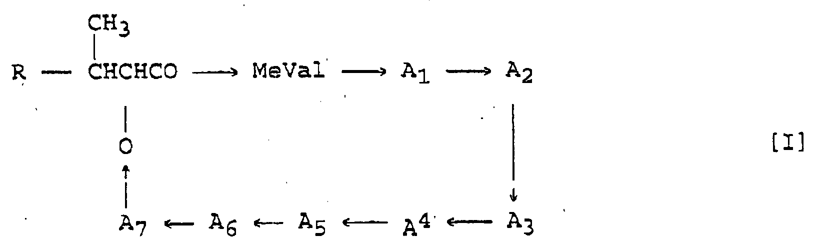 worin
R Methyl oder Ethyl,
A₁ Phe, o-FPhe, m-FPhe oder Tyr,
A₂ MePhe, β-HOMePhe, Phe, o-FMePhe, m-FMePhe, MeTyr, Sar, MeSer, β-oxoMePhe oder β-R₂OMePhe (worin R₂ eine niedere Acylgruppe mit 1 bis 4 Kohlenstoffatomen ist),
A₃ Pro, 4Hyp oder SPro,
A₄ allo-Ile, Val, Leu oder Nle,
A₅ MeVal oder Val,
A₆ Leu oder Nva, und
A₇ β-HoMeVal, γ-HOMeVal, MeVal, N,β-MeAsp, β-HOMePhe, MePhe, MeDH_{2,3}Val, MeDH_{3,4}Val oder Sar sind,
und ein nicht-toxisches oberflächenaktives Mittel.

2. Arzneimittel nach Anspruch 1, dadurch gekennzeichnet, daß das oberflächenaktive Mittel ein nicht-ionisches Mittel ist,

3. Arzneimittel nach Anspruch 2, dadurch gekennzeichnet, daß das nichtionische Mittel Polyoxyethylen-hydriertes Rizinusöl ist.

4. Verfahren zur Herstellung eines Arzneimittels in fester Form, umfassend die Stufe der Zugabe einer pharmakologisch wirksamen Menge von Aureobasidin der allgemeinen Formel [I] 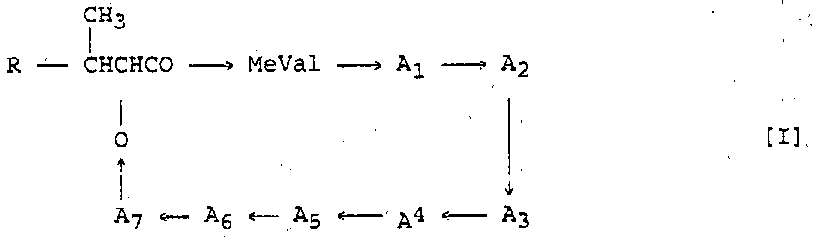 worin
R Methyl oder Ethyl,
A₁ Phe, o-FPhe, m-FPhe oder Tyr,
A₂ MePhe, β-HOMePhe, Phe, o-FMePhe, m-FMePhe, MeTyr, Sar, MeSer, β-oxoMePhe oder β-R₂OMePhe (worin R₂ eine niedere Acylgruppe mit 1 bis 4 Kohlenstoffatomen ist),
A₃ Pro, 4Hyp oder SPro,
A₄ allo-Ile, Val, Leu oder Nle,
A₅ MeVal oder Val,
A₆ Leu oder Nva, und
A₇ β-HOMeVal, γ-HoMeVal, MeVal, N,β-MeAsp, β-HOMePhe, MePhe, MeDH_{2,3}Val, MeDH_{3,4}Val oder Sar sind,
zu einem nicht-toxischen oberflächenaktiven Mittel.

5. Verfahren nach Anspruch 4, dadurch gekennzeichnet, daß das oberflächenaktive Mittel ein nicht-ionisches Mittel ist.

6. Verfahren nach Anspruch 5, dadurch gekennzeichnet, daß das nichtionische Mittel Polyoxyethylen-hydriertes Rizinusöl ist.

## Revendications (Revendications pour l'(les) Etat(s) contractant(s) suivant(s): AT, BE, CH, DE, DK, FR, GB, GR, IT, LI, LU, MC, NL, PT, SE)

1. Composition pharmaceutique sous forme solide, comprenant une quantité pharmacologiquement efficace d'auréobasidine représentée par la formule générale (I) : 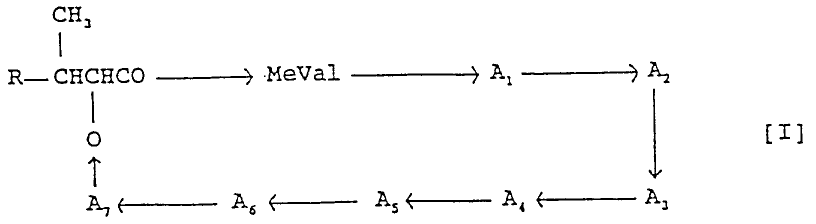 dans laquelle :
R représente un groupe méthyle ou éthyle ;
A₁ représente un groupe Phe, o-FPhe, m-FPhe ou Tyr ;
A₂ représente un groupe MePhe, β-HOMePhe, Phe, o-FMePhe, m-FMePhe, MeTyr, Sar, MeSer, β-oxoMePhe ou β-R₂OMePhe (dans lequel R₂ représente un groupe acyle inférieur ayant 1 à 4 atomes de carbone) ;
A₃ représente un groupe Pro, 4Hyp ou SPro ;
A₄ représente un groupe allo-Ile, Val, Leu ou Nle ;
A₅ représente un groupe MeVal ou Val ;
A₆ représente un groupe Leu ou Nva ; et
A₇ représente un groupe β-HOMeVal, γ-HOMeVal, MeVal, N,β-MeAsp, β-HOMePhe, MePhe, MeDH_{2,3}Val, MeDH_{3,4}Val ou Sar,
et un agent tensioactif non toxique.

2. Composition pharmaceutique suivant la revendication 1, dans laquelle l'agent tensioactif est un agent non ionique.

3. Composition pharmaceutique suivant la revendication 2, dans laquelle l'agent non ionique consiste en polyoxyéthylène-huile de ricin hydrogénée.

## Revendications (Revendications pour l'(les) Etat(s) contractant(s) suivant(s): ES)

1. Composition pharmaceutique sous forme solide, comprenant une quantité pharmacologiquement efficace d'auréobasidine représentée par la formule générale (I) : 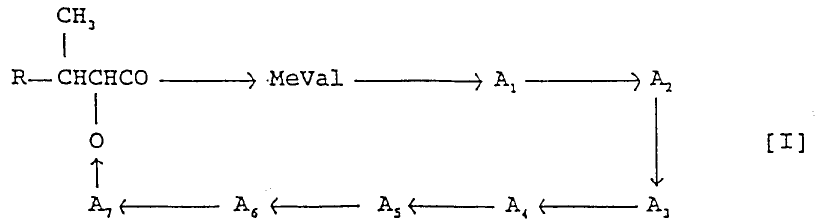 dans laquelle :
R représente un groupe méthyle ou éthyle ;
A₁ représente un groupe Phe, o-FPhe, m-FPhe ou Tyr ;
A₂ représente un groupe MePhe, β-HOMePhe, Phe, o-FMePhe, m-FMePhe, MeTyr, Sar, MeSer, β-oxoMePhe ou β-R₂OMePhe (dans lequel R₂ représente un groupe acyle inférieur ayant 1 à 4 atomes de carbone) ;
A₃ représente un groupe Pro, 4Hyp ou SPro ;
A₄ représente un groupe allo-Ile, Val, Leu ou Nle ;
A₅ représente un groupe MeVal ou Val ;
A₆ représente un groupe Leu ou Nva ; et
A₇ représente un groupe β-HOMeVal, γ-HOMeVal, MeVal, N,β-MeAsp, β-HOMePhe, MePhe, MeDH_{2,3}Val, MeDH_{3,4}Val ou Sar,
et un agent tensioactif non toxique.

2. Composition pharmaceutique suivant la revendication 1, dans laquelle l'agent tensioactif est un agent non ionique.

3. Composition pharmaceutique suivant la revendication 2, dans laquelle l'agent non ionique consiste en Polyoxyéthylène-huile de ricin hydrogénée.

4. Procédé de préparation d'une composition pharmaceutique sous forme solide, comprenant l'étape d'addition d'une quantité, pharmacologiquement efficace, d'auréobasidine représentée par la formule générale (I) : 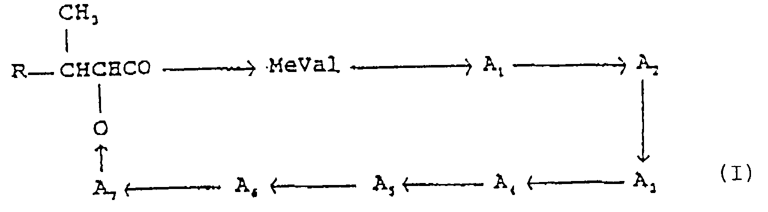 dans laquelle :
R représente un groupe méthyle ou éthyle ;
A₁ représente un groupe Phe, o-FPhe, m-FPhe ou Tyr ;
A₂ représente un groupe MePhe, β-HOMePhe, Phe, o-FMePhe, m-FMePhe, MeTyr, Sar, MeSer, β-oxoMePhe ou β-R₂OMePhe (dans lequel R₂ représente un groupe acyle inférieur ayant 1 à 4 atomes de carbone) ;
A₃ représente un groupe Pro, 4Hyp ou SPro ;
A₄ représente un groupe allo-Ile, Val, Leu ou Nle ;
A₅ représente un groupe MeVal ou Val ;
A₆ représente un groupe Leu ou Nva ; et
A₇ représente un groupe β-HOMeVal, γ-HOMeVal, MeVal, N,β-MeAsp, β-HOMePhe, MePhe, MeDH_{2,3}Val, MeDH_{3,4}Val ou Sar,
à un agent tensioactif non toxique.

5. Procédé suivant la revendication 1, dans lequel l'agent tensioactif est un agent non ionique.

6. Procédé suivant la revendication 2, dans lequel l'agent non ionique consiste en polyoxyéthylène-huile de ricin hydrogénée.
